# FASCICULE DE BREVET EUROPEEN

(11) **EP 0 913 378 B1**
(45) Date de publication et mention de la délivrance du brevet: **26.09.2001**
(21) Numéro de dépôt: 98402677.3
(22) Date de dépôt: 27.10.1998
(51) Int. Cl.: C07C 11/02, C07C 5/333, B01J 23/62

(54) **Procédé de déshydrogénation d'hydrocarbures aliphatiques saturés en hydrocarbures oléfiniques**
Verfahren zur Dehydrierung von gesättigten aliphatischen Kohlenwasserstoffen in olefinischen Kohlenwasserstoffen
Process for the dehydrogenation of saturated aliphatic hydrocarbons in olefinic hydrocarbons

(30) Priorité: 31.10.1997 FR 9713685
(43) Date de publication de la demande: 06.05.1999
(73) Titulaire: INSTITUT FRANCAIS DU PETROLE, 92852 Rueil-Malmaison Cedex (FR)
(72) Inventeur: Le Peltier, Fabienne, 92500 Rueil Malmaison (FR); Didillon, Blaise, 92500 Rueil Malmaison (FR); Clause, Olivier, 78400 Chatou (FR)
(74) Mandataire: Andréeff, François

(56) Documents cités:
- EP-A- 0 623 385

## Description

La présente invention concerne un procédé de déshydrogénation d'une charge d'hydrocarbures aliphatiques en hydrocarbures oléfiniques correspondants, en présence d'un catalyseur renfermant au moins un support, au moins un métal du groupe VIII de la classification périodique des éléments, et au moins un élément additionnel M choisi dans le groupe constitué par le germanium, l'étain, le plomb, le rhénium, le gallium, l'indium et le thallium, ledit procédé étant caractérisé en ce qu'on utilise un catalyseur préparé selon un procédé dans lequel l'élément additionnel M est introduit dans un solvant aqueux sous la forme d'au moins un composé organométallique comprenant au moins une liaison carbone-M.

Plus précisément, la présente invention concerne un nouveau procédé de déshydrogénation d'hydrocarbures aliphatiques C₅-C₂₂ en hydrocarbures oléfiniques correspondants, en présence d'un catalyseur renfermant au moins un support, au moins un métal du groupe VIII de la classification périodique des éléments, et au moins un élément additionnel M choisi dans le groupe constitué par le germanium, l'étain, le plomb, le rhénium, le gallium, l'indium et le thallium. Ce catalyseur peut aussi contenir un autre élément choisi dans le groupe constitué par les métaux alcalins ou alcalino-terreux et/ou un métalloïde tel que le soufre et/ou tout autre élément chimique tel qu'un halogène ou composé halogéné.

Les formulations de catalyseurs utilisés dans les procédés de conversion d'hydrocarbures ont fait l'objet d'un très grand nombre d'études. Les brevets et publications démontrant que l'addition de promoteurs à un métal de base améliore la qualité des catalyseurs sont fort nombreux. Pour les catalyseurs de déshydrogénation de paraffines, on connaît de longue date les catalyseurs renfermant outre un support, un métal noble de la famille du platine et au moins un métal additionnel M (US-3 998 900 et US-3 531 543). L'acidité du support inorganique réfractaire peut conduire à des réactions secondaires indésirables telles que les réactions de craquage et d'isomérisation. C'est pourquoi le support oxyde est généralement neutralisé par l'ajout d'au moins un élément alcalin ou alcalino-terreux.

La phase métallique est la fonction hydro-déshydrogénante qui assure la déshydrogénation des paraffines et l'hydrogénation des précurseurs de coke. Mais le platine présente une activité hydrogénolysante au détriment des rendements en oléfines souhaités dans le procédé de déshydrogénation de paraffines. Cette activité hydrogénolysante peut être fortement réduite, donc la sélectivité du catalyseur augmentée, par l'ajout d'un métal additionnel M. Par ailleurs, l'ajout de cet élément M peut aussi augmenter les propriétés hydrogénantes du platine, ce qui favorise l'hydrogénation des précurseurs de coke et donc la stabilité du catalyseur.

Ces éléments sont ajoutés sous différentes formes telles que sels minéraux ou composés organométalliques. La façon dont ces modificateurs sont introduits n'est pas indifférente car elle conditionne fortement les propriétés du catalyseur. Ainsi l'introduction du métal M est avantageusement effectuée à l'aide d'un composé organométallique dudit métal M. Cette technologie d'introduction du métal M a été décrite dans le brevet US 3 531 543. Le métal M est introduit sous la forme d'au moins un composé organométallique choisi dans le groupe formé par les complexes, en particulier les complexes carbonyles, polycétoniques des métaux M et les hydrocarbyls métaux du métal M tels que les alkyles, les cycloalkyles, les aryles, les akylaryles métaux et les arylalkyles métaux.

Cette introduction de l'élément additionnel M sous la forme d'un composé organométallique conduit à des catalyseurs plus performants mais nécessite l'emploi d'un solvant organique. Le solvant d'imprégnation décrit selon la technologie du brevet US 4 548 918 est choisi dans le groupe constitué par les solvants organiques oxygénés contenant de 2 à 8 atomes de carbone par molécule, et les hydrocarbures paraffiniques, naphténiques ou aromatiques contenant essentiellement de 6 à 15 atomes de carbone par molécule, et les composés organiques oxygénés halogénés contenant de 1 à 15 atomes de carbone par molécule. Ces solvants peuvent être utilisés seuls ou en mélange entre eux.

De même, le brevet européen EP-B1-0 623 385 déposé par le Demanderesse divulgue un catalyseur utilisé dans les réactions de déshydrogénation de paraffines et comprenant au moins un métal du groupe VIII et un métal additionnel M. Le métal additionnel est introduit sous la forme de composés organiques dans un solvent hydrocarboné tel que ceux décrits ci-dessus.

On a maintenant découvert dans la présente invention qu'il est possible de préparer ces catalyseurs particulièrement performants avec introduction du métal M sous la forme d'un complexe organométallique soluble dans un solvant aqueux. Ceci représente un progrès considérable de facilité de mise en oeuvre lors de la fabrication du catalyseur. En effet, l'usage de quantités industrielles de solvants organiques présente d'importants inconvénients en termes de sécurité (inflammabilité, toxicité) et en termes de coûts.

Le support du catalyseur selon l'invention comporte au moins un oxyde réfractaire qui est généralement choisi parmi les oxydes de métaux des groupes IIA, IIIA, IIIB, IVA ou IVB de la classification périodique des éléments tels que par exemple les oxydes de magnésium, d'aluminium, de silicium, de titane, de zirconium, de thorium pris seuls ou en mélange entre eux ou en mélange avec des oxydes d'autres éléments de la classification périodique . On peut aussi utiliser le charbon. On peut aussi utiliser des zéolithes ou tamis moléculaires de type X, Y, mordénite, faujasite, ZSM-5, ZSM-4, ZSM-8, ainsi que les mélanges d'oxydes de métaux des groupes IIA, IIIA, IIIB, IVA et IVB avec du matériau zéolithique.

Le support préféré est l'alumine, dont la surface spécifique est avantageusement comprise entre 5 et 400 m² par gramme, de préférence entre 50 et 350 m² par gramme.

Le catalyseur selon l'invention, renferme outre un support :
a) au moins un métal du groupe VIII choisi parmi l'iridium, le nickel, le palladium, le platine, le rhodium et le ruthénium. Le platine et le palladium sont les métaux préférés. Le pourcentage pondéral est choisi entre 0,1 et 10 % et de préférence entre 0,1 et 5 %.
b) au moins un élément additionnel M choisi dans le groupe constitué par le germanium, l'étain, le plomb, le rhénium, le gallium, l'indium et le thallium. L'étain et le germanium sont les éléments préférés. Le pourcentage pondéral est choisi entre 0,01 et 10 %, et de préférence entre 0,02 et 5 %. On peut avantageusement dans certains cas utiliser à la fois au moins deux des métaux de ce groupe.
c) de manière préférée de 0,1 à 3 % poids d'au moins un métal alcalin ou alcalino-terreux.

Suivant le domaine d'application, le catalyseur peut contenir en plus de 0,01 à 3 % poids d'un halogène ou composé halogéné. Il peut aussi contenir de 0,01 à 2 % poids d'un élément tel que le soufre.

Le catalyseur peut être préparé par différentes procédures d'imprégnation du support et l'invention n'est pas limitée à une procédure d'imprégnation déterminée. Quand on utilise plusieurs solutions, on peut procéder à des séchages et/ou des calcinations intermédiaires.

L'élément additionnel M peut être introduit lors de l'élaboration du support. Une méthode consiste par exemple à malaxer la poudre humide de support avec les précurseurs du catalyseur et à mettre ensuite en forme et sécher.

On peut introduire le métal du groupe VIII, le métal additionnel M, le métal alcalin ou alcalino-terreux, éventuellement l'halogène ou le composé halogéné, éventuellement le métalloïde, simultanément ou successivement, dans n'importe quel ordre. Selon l'invention, la mise en contact de l'élément organométallique M est caractérisée en ce qu'il est introduit dans un solvant aqueux.

Dans une autre méthode, le métal additionnel M, peut-être introduit lors de la synthèse du support selon une technique de type Sol-Gel. Par exemple, pour un support contenant de l'alumine, un gel mixte métal M-alumine peut être obtenu en hydrolysant avec une solution aqueuse d'un composé organométallique du métal M une solution organique de Al(OR')₃ dans un solvant tel ROH ou R'OH. R et R' peuvent désigner un groupement alkyl de type méthyl, éthyl, isopropyl, n-propyl, butyl, voire un groupement plus lourd, tel le n-hexyl. Le solvant alcoolique doit être déshydraté de façon poussée avant l'introduction de l'alcoolate d'aluminium. Après l'hydrolyse, un traitement thermique du gel obtenu opéré à une température comprise entre 200 et 800°C, de préférence entre 300 et 700°C et de manière encore plus préférée entre 400 et 500°C, permet d'assurer la réaction complète du composé organométallique hydrosoluble du métal M avec le gel, ce qui entraîne la formation de l'oxyde mixte Al2O₃-MOₓ.

Dans une autre méthode le métal M peut être ajouté à un sol d'alumine. Le brevet US-3 929 683 décrit l'introduction de l'étain sous forme de sel, par exemple SnCl₂ dans un sol d'alumine. Selon la présente invention, il est possible d'ajouter un composé organométallique hydrosoluble de métal M à un hydrosol d'alumine, obtenu par exemple en précipitant à pH 4-5 une solution acide de AlCl₃, puis de favoriser la réaction du composé du métal M avec l'hydrosol d'alumine, par exemple sous l'effet de la chaleur ou d'une base.

Le précurseur de l'élément M peut être choisi dans le groupe des composés halogénés, des hydroxydes, des oxydes, des carbonates, et des carboxylates de composés organométalliques de l'élément M. Ces composés comprennent au moins une liaison carbone-M. Le précurseur de l'élément M peut être aussi choisi parmi les composés de formule générale (R1)ₓM(R2)_{y} avec x+y=valence du métal M et où R1 est choisi dans le groupe des fonctions alkyles, cycloalkyles, aryles, alkylaryles et arylalkyles et R2 est une fonction de la forme CₐH_{b}R'_{c} où R' représente une fonction hydroxyde, carboxylate, PO₃H ou SO₃H.

Dans une technique de préparation selon l'invention, le catalyseur est obtenu par imprégnation du support, à l'aide d'une solution aqueuse ou organique d'au moins un élément alcalin ou alcalino-terreux, le volume de la solution étant de préférence égal au volume de rétention du support ou en excès par rapport à ce volume. Le support imprégné est ensuite filtré, séché puis calciné sous air habituellement entre 110 et environ 550°C. Ensuite on introduit au moins un composé de métal du groupe VIII, à l'aide d'une solution aqueuse ou organique, le volume de la solution étant de préférence en excès par rapport au volume de rétention du support ou égal à ce volume. Le support imprégné est ensuite filtré, puis séché et calciné sous air habituellement entre 110 et environ 550°C, puis ensuite réduit sous hydrogène à une température habituellement comprise entre environ 200 et environ 600°C et de préférence entre environ 300 et environ 500°C. Le produit obtenu est alors imprégné par une solution aqueuse d'un composé d'étain, de germanium, de plomb, de rhénium, de gallium, d'indium ou de thallium. D'une manière particulièrement avantageuse, on utilise une solution aqueuse d'un composé carboxylate d'étain, par exemple l'acétate de tributyl étain.

Le volume de la solution aqueuse est de préférence égal au volume de rétention du support et de manière encore plus préférée en excès par rapport à ce volume. La valeur de la concentration en au moins un métal M dans la solution aqueuse est choisie avantageusement entre 0,01 et 25 mmol/l et de manière préférée entre 0,5 et 20 mmol/l et de manière encore plus préférée entre 0,5 et 15 mmol/l. La valeur de pH de la solution est choisie avantageusement entre 10 et 14 et de manière préférée entre 10 et 12.

Après avoir laissé le contact entre le support imprégné du métal du groupe VIII et la solution contenant au moins un composé de l'élément M pendant plusieurs heures, le produit est filtré, éventuellement lavé à l'eau puis séché. On termine, selon cette méthode, par une réduction entre 300 et 600°C, de préférence en effectuant un balayage sous hydrogène pendant plusieurs heures.

Dans une autre technique selon l'invention, le catalyseur est obtenu par imprégnation d'une solution aqueuse d'au moins un composé dudit métal M, le volume de la solution étant de préférence égal au volume de rétention du support et de manière encore plus préférée en excès par rapport à ce volume. D'une manière particulièrement avantageuse, on utilise une solution aqueuse d'un composé carboxylate d'étain. La valeur de la concentration en au moins un métal M dans la solution aqueuse est choisie avantageusement entre 0,01 et 25 mmol/l et de manière préférée entre 0,5 et 20 mmol/l et de manière encore plus préférée entre 0,5 et 15 mmol/l. La valeur de pH de la solution est choisie avantageusement entre 10 et 14 et de manière préférée entre 10 et 12. Après avoir laissé le contact entre le solide et la solution d'imprégnation pendant plusieurs heures, le produit est ensuite séché. On termine habituellement par une calcination entre 300 et 600°C, de préférence en effectuant un balayage d'air durant plusieurs heures. Le solide obtenu peut être alors imprégné par une solution aqueuse ou organique d'au moins un composé alcalin ou alcalino-terreux, le volume de la solution étant de préférence égal au volume de rétention du support ou en excès par rapport à ce volume. Le support imprégné est ensuite séché puis calciné sous air habituellement entre 110 et environ 550°C. On peut aussi et de manière avantageuse, introduire simultanément au moins un composé dudit élément M et au moins un élément alcalin ou alcalino-terreux, par une imprégnation du support par une solution aqueuse commune, le volume de la solution étant de préférence égal au volume de rétention du support ou en excès par rapport à ce volume. Après avoir laissé le contact entre le solide et la solution d'imprégnation pendant plusieurs heures, le produit est ensuite séché. On termine habituellement par une calcination entre 300 et 600°C, de préférence en effectuant un balayage d'air durant plusieurs heures. Le solide obtenu est ensuite imprégné à l'aide d'une solution aqueuse ou organique d'au moins un composé de métal du groupe VIII, le volume de la solution étant de préférence en excès par rapport au volume de rétention du support ou égal à ce volume. Après quelques heures de mise en contact, le produit obtenu est ensuite séché puis calciné sous air entre 300 et 600°C. de préférence en effectuant un balayage d'air durant plusieurs heures.

Avant utilisation on réduit le catalyseur sous hydrogène par exemple entre 20 et 600°C afin d'obtenir une phase métallique active. La procédure de ce traitement consiste par exemple en une montée lente de la température sous courant d'hydrogène jusqu'à la température maximale de réduction, comprise par exemple entre 20 et 600°C, et de préférence entre 90 et 500°C, suivie d'un maintien pendant par exemple durant 1 à 6 heures à cette température.

Cette réduction pouvant être effectuée aussitôt après la calcination, ou plus tard chez l'utilisateur. Il est aussi possible de réduire directement le produits séché directement chez l'utilisateur.

Il est aussi possible d'effectuer la réduction préalable du composé de métal du groupe VIII en solution par des molécules organiques à caractère réducteur telles que l'acide formique. On peut alors introduire le composé de l'élément additionnel M simultanément ou successivement. Une possibilité consiste à filtrer, puis sécher le catalyseur obtenu. Il peut être alors calciné puis réduit dans les conditions décrites ci-dessus. Il est aussi possible d'effectuer la réduction directement à partir du produit séché.

Selon l'invention, le catalyseur décrit précédemment est mis en oeuvre dans les procédés de déshydrogénation de paraffines C₅-C₂₂. Les procédés de déshydrogénation de paraffines légères C₅ permet de valoriser des hydrocarbures aliphatiques à bas point d'ébullition tels que les pentanes et isopentanes que l'on peut récupérer après extraction des insaturés des coupes C₅ de vapocraquage ou de craquage catalytique. Le procédé de déshydrogénation de paraffines plus longues est un procédé commercial important du fait de la demande actuelle en monooléfines pour la préparation de détergents biodégradables ou de produits pharmaceutiques par exemple.

Ces différents procédés se différencient par le choix des conditions opératoires et de la composition de la charge. L'ajustement des conditions opératoires, en fonction de la nature de la charge à traiter, se fait de façon à obtenir la meilleure adéquation pression-température-rendement et activité de façon connue de l'homme du métier. La réaction de déshydrogénation s'effectue en général à une pression comprise entre 0.2 et 20 bars absolus sous une pression préférée de 1 à 10 bars absolus et à une température comprise entre 400 et 800°C en fonction de la nature de la charge.

La température est avantageusement comprise entre 400 et 550°C pour une charge comprenant essentiellement de l'isopentane. La température est avantageusement comprise entre 450 et 550°C pour une charge comprenant principalement des paraffines comportant de 9 à 22 atomes de carbone par molécule. La charge peut aussi contenir des hydrocarbures insaturés comportant de 5 à 22 atomes de carbone par molécule. Le débit massique de charge traitée par unité de masse de catalyseur est généralement compris entre 0.5 à 100 kg/kg/h. Il peut être avantageux d'utiliser l'hydrogène comme diluant. Le rapport molaire hydrogène/hydrocarbure est généralement compris entre 0 et 20, de préférence entre 0 et 6.

Les exemples qui suivent illustrent l'invention sans toutefois en limiter la portée.

### EXEMPLE 1

On prépare deux catalyseurs A et B renfermant 0.3 % poids de platine, 0.8 poids d'étain et 0.7 % poids de lithium. Le support est une alumine γ de surface spécifique de 210 m² par gramme.

### Catalyseur A (comparatif)

Le catalyseur A est préparé selon les techniques de l'art antérieur. A 100 g de support alumine on ajoute 60 cm³ d'une solution aqueuse d'acétate de lithium contenant 0.7 g de lithium. On laisse en contact 3 heures puis le solide est séché 1 heure à 120°C puis calciné à 350 °C durant deux heures. On met alors le solide en contact avec 60 cm³ d'un solution aqueuse d'acétate d'étain contenant 0.8 g d'étain. Après 3 heures de mise en contact, le solide est séché 1 heure à 120°C puis calciné 2 heures à 530°C. Ensuite on procède à l'introduction du platine par ajout de 400 cm³ d'une solution de toluène contenant 0.3 g de platine sous la forme d'acétylacétonate de platine. On laisse 24 heures en contact puis on sèche 1 heure à 120°C et on calcine 2 heures à 530°C.

### Catalyseur B (selon l'invention)

A 100 g de support alumine on ajoute 60 cm³ d'une solution aqueuse d'acétate de lithium contenant 0.7 g de lithium. On laisse en contact 3 heures puis le solide est séché 1 heure à 120°C puis calciné à 350°C durant deux heures. Ensuite on procède directement à l'introduction du platine par ajout de 400 cm³ d'une solution de toluène contenant 0.3 g de platine sous la forme d'acétylacétonate de platine. On laisse 24 heures en contact puis on filtre, on sèche 1 heure à 120°C et on calcine 2 heures à 530°C. Le solide est ensuite réduit à 450°C sous hydrogène, et introduit sans remise à l'air, dans un réacteur contenant 400 cm³ une solution aqueuse ammoniacale (pH 10) contenant 0.8 g d'étain sous la forme de tributyl acétate d'étain (Bu₃SnOC(O)CH₃). Après 24 heures de mise en contact, le mélange réactionnel est filtré, lavé, séché et réduit à 450°C.

### EXEMPLE 2

Les catalyseurs A et B sont soumis à un test de déshydrogénation du n-dodécane réalisé dans un réacteur tubulaire isotherme. 2 g de catalyseur sont réduits à 450 °C durant 2 heures sous un débit de 4 litres par heure d'hydrogène. Les conditions opératoires sont les suivantes :
- charge : n-dodécane
- température : 450 ou 470 °C
- pression : 0.2 MPa
- H₂/nC₁₂ (molaire) : 5
- débit massique nC₁₂ liquide/masse de catalyseur : 80 h⁻¹

Les résultats obtenus dans ces conditions sont rapportés dans le tableau 1. Les valeurs de conversion de nC₁₂ et de rendements sont exprimés en % poids par rapport à la charge.

**Tableau 1**

| Catalyseurs | Température | conversion (%) | rendements (%) | |
|---|---|---|---|---|
| | (°C) | nC₁₂ | oléfines nC₁₂ | aromatiques |
| A | 450 | 10.4 | 9.6 | 0.3 |
| | 470 | 12.1 | 10.3 | 0.8 |
| | 450 | 6.0 | 5.6 | 0.1 |
| | | | | |
| B | 450 | 10.6 | 9.7 | 0.2 |
| | 470 | 15.0 | 13.2 | 0.6 |
| | 450 | 9.6 | 8.4 | 0.2 |

Le catalyseur B préparé selon l'invention, en milieu aqueux à partir d'un précurseur Sn organométallique, présente des rendements en oléfines supérieurs à ceux du catalyseur A préparé en phase aqueuse à partir d'un composé Sn minéral. De plus comme l'indiquent les résultats catalytiques correspondant au « point retour» à 450 °C, le catalyseur B présente une meilleure stabilité que le catalyseur A.

## Revendications

1. Procédé de déshydrogénation d'une charge d'hydrocarbures aliphatiques en hydrocarbures oléfiniques correspondants, en présence d'un catalyseur renfermant au moins un support, au moins un métal du groupe VIII de la classification périodique des éléments, et au moins un élément additionnel M choisi dans le groupe constitué par le germanium, l'étain, le plomb, le rhénium, le gallium, l'indium et le thallium, ledit procédé étant **caractérisé en ce qu'**on utilise un catalyseur préparé selon un procédé dans lequel l'élément additionnel M est introduit dans un solvant aqueux sous la forme d'au moins un composé organométallique comprenant au moins une liaison carbone-M.

2. Procédé de déshydrogénation d'hydrocarbures aliphatiques en hydrocarbures oléfiniques correspondants selon la revendication 1, **caractérisé en ce que** la charge est constituée d'hydrocarbures aliphatiques C5-C22.

3. Procédé selon la revendication 1 à 2 tel que le catalyseur contient en outre au moins un métal alcalin ou alcalino-terreux.

4. Procédé selon l'une des revendications 1 à 3 tel que le catalyseur contient en outre au moins un métalloïde.

5. Procédé selon l'une des revendications 1 à 4 tel que le catalyseur contient en outre au moins un halogène ou un composé halogéné.

6. Procédé selon l'une des revendications 1 à 5 tel que dans le catalyseur, le métal du groupe VIII est choisi parmi l'iridium, le nickel, le palladium, le platine, le rhodium et le ruthénium, de préférence le platine et le palladium.

7. Procédé selon l'une des revendications 1 à 6 tel que dans le catalyseur l'élément M est choisi parmi le germanium et l'étain.

8. Procédé selon l'une des revendications 1 à 7 tel que dans le catalyseur le précurseur de l'élément M est choisi dans le groupe des hydroxydes, des composés halogénés, et des carboxylates de composés organiques de l'élément M, les composés de formules générales (R1)ₓ M (R2)_{y} avec x+y=valence du métal M et où R1 est choisi dans le groupe des fonctions alkyles, cycloalkyles, aryles, alkylaryles et arylalkyles et R2 est une fonction de la forme CₐH_{b}R'_{c} où R' représente une fonction hydroxyde, carboxylate, PO₃H ou SO₃H.

9. Procédé selon l'une des revendications 1 à 8 tel que dans le catalyseur le précurseur de l'élément M est choisi dans le groupe des carboxylates de composés organiques de l'élément M.

10. Procédé selon l'une des revendications 1 à 9 tel que dans le catalyseur le précurseur de l'élément M est l'acétate de tributyl étain.

11. Procédé selon l'une des revendications 1 à 10 tel que lors de la préparation du catalyseur, le métal du groupe VIII, l'élément additionnel M, le métal alcalin ou alcalino-terreux, éventuellement l'halogène, éventuellement le métalloïde, sont introduits successivement ou simultanément sur le support.

12. Procédé selon l'une des revendications 1 à 11 tel que l'on prépare le catalyseur selon les étapes, dans n'importe quel ordre :
• on imprègne un support à l'aide d'une solution aqueuse ou organique d'au moins un métal alcalin ou alcalino-terreux, on filtre, on sèche, on calcine,
• on imprègne à l'aide d'une solution aqueuse ou organique d'au moins un métal du groupe VIII, on filtre, on sèche, on calcine, on réduit,
• on imprègne le produit obtenu par une solution aqueuse d'un composé de l'élément M additionnel, on filtre, on sèche, on réduit éventuellement, on calcine.

13. Procédé selon l'une des revendications 1 à 12 tel que lors de la préparation du catalyseur, on imprègne le support avec une solution aqueuse d'au moins un métal M, le volume de la solution étant au moins égal au volume de rétention du support, et de préférence supérieur à ce volume.

14. Procédé selon l'une des revendications 1 à 13 tel que lors de la préparation du catalyseur, la concentration en au moins un métal M dans la solution aqueuse est comprise entre 0,01 et 25 mmol/l.

15. Procédé selon la revendication 14 tel que la concentration en au moins un métal M dans la solution aqueuse est comprise entre 0,5 et 20 mmol/l et de préférence entre 0,5 et 15 mmol/l.

16. Procédé selon l'une des revendications 1 à 15 tel que lors de la préparation du catalyseur, la valeur de pH de la solution aqueuse d'au moins un composé du métal M est choisie entre 10 et 14 et de manière préférée entre 10 et 12.

17. Procédé selon l'une des revendications 1 à 11 tel que dans la préparation du catalyseur on introduit l'élément additionnel M lors de l'élaboration du support.

18. Procédé selon la revendication 17 tel que dans la préparation du catalyseur l'élément additionnel M est introduit lors de la synthèse du support selon une technique de type SOL-GEL.

19. Procédé selon la revendications 18 tel que dans la préparation du catalyseur on hydrolyse avec une solution aqueuse d'un composé organométallique de métal M une solution organique d'un composé alcoxy d'un métal du support dans un solvant alcoolique, et on chauffe à une température comprise entre 200 et 800°C.

20. Procédé selon l'une des revendications 1 à 19 tel que le catalyseur est réduit sous hydrogène à une température comprise entre 20 et 600°C, de préférence entre 90 et 500°C.

21. Procédé selon l'une des revendications 1 à 20 tel que l'on effectue une réduction préalable du composé du métal VIII en solution par des molécules organiques à caractère réducteur tel que l'acide formique.

22. Procédé selon l'une des revendications 1 à 21 dans la charge à traiter est mise en contact avec le catalyseur sous une pression comprise entre 0,2 et 20 bars absolus et à une température comprise entre 400 et 800°C, et avec un débit massique de charge traitée par unité de masse de catalyseur compris entre 0,5 et 100 kg/kg/heure.

23. Procédé selon l'une des revendications 1 à 22 tel que la température est comprise entre 400 et 550°C pour une charge contenant de l'isopentane.

24. Procédé selon l'une des revendications 1 à 23 tel que la température est comprise entre 450 et 550 pour une charge contenant de 9 à 22 atomes de carbone.

25. Procédé selon l'une des revendications 1 à 24 tel que l'hydrogène est utilisé comme diluant des hydrocarbures, le rapport molaire hydrogène/hydrocarbures étant compris entre 0 et 20.

## Patentansprüche

1. Dehydrierungsverfahren einer Charge von aliphatischen Kohlenwasserstoffen zu entsprechenden olefinischen Kohlenwasserstoffen in Gegenwart eines Katalysators, der wenigstens einen Träger, wenigstens ein Metall der Gruppe VIII des Periodensystems der Elemente und wenigstens ein zusätzliches Element M einschließt, das in der durch Germanium, Zinn, Blei, Rhenium, Gallium, Indium und Thallium gebildeten Gruppe gewählt ist, wobei das Verfahren **dadurch gekennzeichnet ist, dass** man einen Katalysator verwendet, der gemäß einem Verfahren hergestellt ist, in dem das zusätzliche Element M in einem wässrigen Lösungsmittel in Form wenigstens einer organo-metallischen Verbindung eingeführt wird, die wenigstens eine Kohlenstoff-M-Bindung umfasst.

2. Dehydrierungsverfahren von aliphatischen Kohlenwasserstoffen zu entsprechenden olefinischen Kohlenwasserstoffen nach Anspruch 1, **dadurch gekennzeichnet, dass** die Charge von aliphatischen C5-C22-Kohlenwasserstoffen gebildet wird.

3. Verfahren nach Anspruch 1 bis 2, derart, dass der Katalysator im übrigen wenigstens ein Alkali- oder Erdalkalimetall enthält.

4. Verfahren nach einem der Ansprüche 1 bis 3, derart, dass der Katalysator im übrigen wenigstens ein Nichtmetall bzw. Metalloid enthält.

5. Verfahren nach einem der Ansprüche 1 bis 4, derart, dass der Katalysator im übrigen wenigstens ein Halogen oder eine halogenhaltige Verbindung enthält.

6. Verfahren nach einem der Ansprüche 1 bis 5, derart, dass in dem Katalysator das Metall der Gruppe VIII unter Iridium, Nickel, Palladium, Platin, Rhodium und Ruthenium, vorzugsweise Platin und Palladium gewählt ist.

7. Verfahren nach einem der Ansprüche 1 bis 6, derart, dass in dem Katalysator das Element M unter Germanium und Zinn gewählt ist.

8. Verfahren nach einem der Ansprüche 1 bis 7, derart, dass in dem Katalysator der Vorläufer des Elements M in der Gruppe der Hydroxide, der halogenhaltigen Verbindungen und der Carboxylate von organischen Verbindungen des Elements M, den Verbindungen der allgemeinen Formeln (R1)ₓ M(R2)_{y} gewählt ist, mit x + y = Valenz des Metalls M und wo R1 in der Gruppe der Alkyl-, Cycloalkyl-, Aryl-, Alkylaryl- und Arylalkyl-Funktionen gewählt ist und R2 eine Funktion der Form CₐH_{b}R'_{c} ist, wobei R' eine Hydroxid-, Carboxylat-, PO₃H- oder SO₃H-Funktion darstellt.

9. Verfahren nach einem der Ansprüche 1 bis 8, derart, dass in dem Katalysator der Vorläufer des Elements M in der Gruppe der Carboxylate von organischen Verbindungen des Elements M gewählt ist.

10. Verfahren nach einem der Ansprüche 1 bis 9, derart, dass in dem Katalysator der Vorläufer des Elements M Tributylzinnacetat ist.

11. Verfahren nach einem der Ansprüche 1 bis 10, derart, dass während der Herstellung des Katalysators das Metall der Gruppe VIII, das zusätzliche Element M, das Alkali- oder Erdalkalimetall, ggf. das Halogen, ggf. das Nichtmetall bzw. Metalloid nacheinander oder gleichzeitig auf dem Träger eingeführt werden.

12. Verfahren nach einem der Ansprüche 1 bis 11, derart, dass man den Katalysator, in welcher Reihenfolge auch immer, gemäß den Stufen herstellt:
- man imprägniert einen Träger mit Hilfe einer wässrigen oder organischen Lösung von wenigstens einem Alkalioder Erdalkalimetall, man filtriert, man trocknet, man kalziniert,
- man imprägniert mit Hilfe einer wässrigen oder organischen Lösung von wenigstens einem Metall der Gruppe VIII, man filtriert, man trocknet, man kalziniert, man reduziert,
- man imprägniert das erhaltene Produkt durch eine wässrige Lösung einer Verbindung des zusätzlichen Elements M, man filtriert, man trocknet, man reduziert ggf., man kalziniert.

13. Verfahren nach einem der Ansprüche 1 bis 12, derart, dass man während der Herstellung des Katalysators den Träger mit einer wässrigen Lösung von wenigstens einem Metall M imprägniert, wobei das Volumen der Lösung wenigstens gleich dem Durchdringungsvolumen des Trägers und vorzugsweise größer als dieses Volumen ist.

14. Verfahren nach einem der Ansprüche 1 bis 13, derart, dass bei der Herstellung des Katalysators die Konzentration an wenigstens einem Metall M in der wässrigen Lösung zwischen 0,01 und 25 mmol/l liegt.

15. Verfahren nach Anspruch 14, derart, dass die Konzentration an wenigstens einem Metall M in der wässrigen Lösung zwischen 0,5 und 20 mmol/l und vorzugsweise zwischen 0,5 und 15 mmol/l liegt.

16. Verfahren nach einem der Ansprüche 1 bis 15, derart, dass bei der Herstellung des Katalysators der pH-Wert der wässrigen Lösung wenigstens einer Verbindung des Metalls M zwischen 10 und 14 und in bevorzugter Weise zwischen 10 und 12 gewählt wird.

17. Verfahren nach einem der Ansprüche 1 bis 11, derart, dass man in der Herstellung des Katalysators das zusätzliche Element M während der Ausarbeitung des Trägers einführt.

18. Verfahren nach Anspruch 17, derart, dass in der Herstellung des Katalysators das zusätzliche Element M während der Synthese des Trägers gemäß einer Technik vom Typ SOL-GEL eingeführt wird.

19. Verfahren nach Anspruch 18, derart, dass man in der Herstellung des Katalysätors mit einer wässrigen Lösung einer organo-metallischen Verbindung des Metalls M eine organische Lösung einer Alkoxyverbindung eines Metalls des Trägers in einem alkoholischen Lösungsmittel hydrolysiert und man auf eine Temperatur erhitzt, die zwischen 200 und 800°C liegt.

20. Verfahren nach einem der Ansprüche 1 bis 19, derart, dass der Katalysator unter Wasserstoff bei einer Temperatur reduziert wird, die zwischen 20 und 600 °C, vorzugsweise zwischen 90 und 500 °C liegt.

21. Verfahren nach einem der Ansprüche 1 bis 20, derart, dass man eine Reduktion vor der Verbindung des Metalls VIII in Lösung durch organische Moleküle mit reduzierendem Charakter wie Ameisensäure durchführt.

22. Verfahren nach einem der Ansprüche 1 bis 21, in dem die zu behandelnde Charge mit dem Katalysator unter einem Druck in Kontakt gebracht wird, der zwischen 0,2 und 20 bar absolut liegt, und bei einer Temperatur, die zwischen 400 und 800 °C liegt und mit einem Massedurchsatz an zu behandelnder Charge pro Katalysatormasseeinheit, der zwischen 0,5 und 100 kg/kg/h liegt.

23. Verfahren nach einem der Ansprüche 1 bis 22, derart, dass die Temperatur wischen 400 und 550 °C für eine Isopentan enthaltende Charge liegt.

24. Verfahren nach einem der Ansprüche 1 bis 23, derart, dass die Temperatur zwischen 450 und 550 °C für eine Charge, die von 9 bis 22 Kohlenstoffatome enthält, liegt.

25. Verfahren nach einem der Ansprüche 1 bis 24, derart, dass Wasserstoff als Verdünnungsmittel der Kohlenwasserstoffe verwendet wird, wobei das molare Verhältnis Wasserstoff/Kohlenwasserstoffe zwischen 0 und 20 liegt.

## Claims

1. A process for dehydrogenating an aliphatic hydrocarbon feed to the corresponding olefinic hydrocarbons in the presence of a catalyst comprising at least one support, at least one metal from group VIII of the periodic table and at least one additional element M selected from the group formed by germanium, tin, lead, rhenium, gallium, indium and thallium, said process being **characterized in that** the catalyst is prepared using a process in which said additional element M is introduced in an aqueous solvent in the form of at least one organometallic compound containing at least one carbon-M bond.

2. A process for dehydrogenating aliphatic hydrocarbons to the corresponding olefinic hydrocarbons according to claim 1, **characterized in that** the feed is constituted by C₅-C₂₂ aliphatic hydrocarbons.

3. A process according to claim 1 or claim 2, in which the catalyst further contains at least one alkali or alkaline-earth metal.

4. A process according to any one of claims 1 to 3, in which the catalyst further contains at least one metalloid.

5. A process according to any one of claims 1 to 4, in which the catalyst further contains at least one halogen or halogen-containing compound.

6. A process according to any one of claims 1 to 5 in which in the catalyst, the group VIII metal is selected from iridium, nickel, palladium, platinum, rhodium and ruthenium, preferably from platinum and palladium.

7. A process according to any one of claims 1 to 6 in which in the catalyst, element M is selected from germanium and tin.

8. A process according to any one of claims 1 to 7 in which in the catalyst, the precursor of element M is selected from the group formed by hydroxides, halogen-containing compounds, and carboxylates of organic compounds of element M, compounds with general formula (R₁)ₓM(R₂)_{y} where x+y = the valency of metal M and where R₁ is selected from the group formed by alkyl, cycloalkyl, aryl, alkylaryl and arylalkyl functions, and R₂ is a function with formula CₐH_{b}R'_{c}, where R' represents a hydroxide, carboxylate, PO₃H or SO₃H function.

9. A process according to any one of claims 1 to 8 in which in the catalyst, the precursor of element M is selected from the group formed by carboxylates of organic compounds of element M.

10. A process according to any one of claims 1 to 9 in which in the catalyst, the precursor of element M is tributyltin acetate.

11. A process according to any one of claims 1 to 10, in which during preparation of the catalyst, the group VIII metal, additional element M, alkali or alkaline-earth metal, optional halogen and optional metalloid are introduced into the support successively or simultaneously

12. A process according to any one of claims 1 to 11, in which the catalyst is prepared by carrying out the following steps in any order:
• impregnating a support using an aqueous or organic solution of at least one group VIII metal, filtering, drying and calcining;
• impregnating using an aqueous or organic solution of at least one alkali or alkaline-earth metal, filtering, drying, calcining and reducing;
• impregnating the product obtained using an aqueous solution of at least one compound of additional element M, filtering, drying, optionally reducing, then calcining.

13. A process according to any one of claims 1 to 12, in which during preparation of the catalyst, the support is impregnated with an aqueous solution of at least one metal M, the volume of the solution being at least equal to the retention volume of the support, preferably higher than that volume.

14. A process according to any one of claims 1 to 13, in which during preparation of the catalyst, the concentration of at least one metal M in the aqueous solution is in the range 0.01 to 25 mmol/l.

15. A process according to claim 14, in which the concentration of at least one metal M in the aqueous solution is in the range 0.5 to 20 mmol/l, preferably in the range 0.5 to 15 mmol/l.

16. A process according to any one of claims 1 to 15, in which during preparation of the catalyst, the pH of the aqueous solution of at least one compound of metal M is selected so as to be between 10 and 14, preferably between 10 and 12.

17. A process according to any one of claims 1 to 11, in which during preparation of the catalyst, additional element M is introduced during production of the support.

18. A process according to claim 17, in which during preparation of the catalyst, the additional element M is introduced during synthesis of the support using a sol-gel type technique.

19. A process according to claim 18, in which during preparation of the catalyst, an aqueous solution of an organometallic compound of metal M is used to hydrolyse an organic solution of an alkoxy compound of a metal of the support in an alcoholic solvent, and heated to a temperature in the range 200°C to 800°C.

20. A process according to any one of claims 1 to 19, in which the catalyst is reduced in hydrogen at a temperature in the range 20°C to 600°C, preferably in the range 90°C to 500°C.

21. A process according to any one of claims 1 to 20, in which prior reduction of the group VIII metal compound is carried out in solution by organic molecules with a reducing nature such as formic acid.

22. A process according to any one of claims 1 to 21, in which the feed to be treated is brought into contact with the catalyst at a pressure in the range 0.2 to 20 bars absolute and at a temperature in the range 400°C to 800°C, with a mass flow rate of treated feed per unit mass of catalyst in the range 0.5 to 100 kg/kg/hour.

23. A process according to any one of claims 1 to 22, in which the temperature is in the range 400°C to 550°C for a feed comprising isopentane.

24. A process according to any one of claims 1 to 23, in which the temperature is in the range 450°C to 550°C for a feed containing 9 to 22 carbon atoms.

25. A process according to any one of claims 1 to 24, in which hydrogen is used to dilute the hydrocarbons, the hydrogen/hydrocarbon molar ratio being in the range 0 to 20.
